# FASCICULE DE BREVET EUROPEEN

(11) **EP 0 684 314 B1**
(45) Date de publication et mention de la délivrance du brevet: **04.10.2001**
(21) Numéro de dépôt: 94401033.9
(22) Date de dépôt: 10.05.1994
(51) Int. Cl.: C12P 7/44

(54) **Procédé de production par fermentation d'acide itaconique**
Verfahren zur fermentativen Herstellung von Itaconsäure
Process for the production of itaconic acid by fermentation

(43) Date de publication de la demande: 29.11.1995
(73) Titulaire: RHODIA CHIMIE, 92408 Courbevoie Cédex (FR)
(72) Inventeur: Jarry, Alain, F-79500 Maisonnay (FR); Seraudie, Yolaine, F-79500 Melle (FR)
(74) Mandataire: Fabre, Madeleine-France

(56) Documents cités:
- EP-A- 0 341 112
- FR-A- 1 327 937

## Description

La présente invention a pour objet un procédé de préparation d'acide itaconique par fermentation.

La production d'acides organiques par fermentation de sucres, en présence d'un microorganisme approprié, est universellement connue. Des acides typiques de telles fermentations sont notamment l'acide acétique, l'acide lactique, l'acide citrique, l'acide fumarique et plus particulièrement l'acide itaconique.

L'acide itaconique est un acide dicarboxylique présentant la formule suivante:

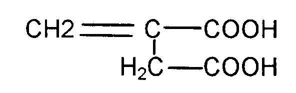

En raison de cette insaturation, les esters d'acide itaconique polymérisent très facilement et sont à ce titre largement utilisés dans les industries des plastiques synthétiques, de la détergence et dans la préparation des adhésifs.

Classiquement, la production de cet acide itaconique est réalisée par fermentation. Diverses variétés d'Aspergillus, et plus particulièrement celles d'Aspergillus terreus et d'Aspergillus itaconicus, sont capables de produire l'acide itaconique par fermentation de carbohydrates.

Les carbohydrates les plus utilisés incluent les mono- et di-saccharides comme le glucose, saccharose, fructose, les amidons dans la mesure où ils se trouvent sous une forme assimilable par le microorganisme, et les mélasses.

FR-A-1 327 937 décrit un procédé pour la production de l'acide itaconique dans lequel la fermentation est conduite en présence de mélasses de betterave comme source d'hydrate de carbone.

Le procédé de production d'acide itaconique décrit dans EP-A-0 341 112 utilise l'amidon comme source de carbone.

En fait, dans les fermentations classiques, pour des impératifs économiques, le choix du substrat hydrocarboné devrait être basé à la fois sur son coût, sur sa disponibilité et son aptitude à conduire à des productivités élevées.

Ceci revient à dire que pour optimiser au niveau économique les procédés de préparations d'acide itaconique, il faut sélectionner une source hydrocarbonée satisfaisant simultanément aux trois exigences énoncées précédemment.

La présente invention a précisément pour objet de proposer une nouvelle source carbonée qui soit disponible en grande quantité, à moindre coût et totalement assimilable par les micro-organismes appropriés à la production d'acide itaconique.

Plus particulièrement, la présente invention concerne un procédé de production d'acide itaconique et/ou d'un de sels par fermentation aérobie d'un carbohydrate au moyen d'un micro-organisme caractérisé en ce que le micro-organisme est une souche d'Aspergillus terreus ou d'Aspergillus itaconicus, et en ce ledit procédé met en oeuvre à titre de substrat carboné, unique ou non, une quantité efficace de glycérol.

De manière inattendue, le glycérol s'est avéré assimilable par les microorganismes appropriés à la production d'acide itaconique. Comparativement aux autres substrats carbonés, énumérés précédemment, le glycérol présente le net avantage d'être particulièrement intéressant sur un plan économique. Il est disponible en grande quantité et à un coût très réduit. Sa mise en oeuvre à titre de substrat carboné conduit à des rendements en acide itaconique importants avec des temps de fermentation comparables à ceux observés avec les substrats carbonés classiques.

Bien entendu, le giycerol peut être utilisé en mélange ou non avec au moins un autre carbohydrate. Celui-ci peut être choisi parmi les mono et di-saccharides tels que le glucose, le saccharose, le fructose, les amidons et les mélasses.

Dans ce type de mélange, les proportions respectives en glycérol et carbohydrate(s) complémentaire(s) peuvent varier largement dans la mesure où leur quantité globale est suffisante pour permettre un déroulement efficace du processus de fermentation.

Pour la formulation des milieux nutritifs correspondants, on met en oeuvre selon l'invention préférentiellement une concentration en substrat carboné, constitué par du glycérol, en mélange ou non avec un ou plusieurs autres carbohydrates complémentaires, variant entre 10 et 240 g/l exprimée en poids par volume.

Selon un mode de réalisation particulier de l'invention, il est possible de neutraliser partiellement, au cours du processus de fermentation, l'acide itaconique produit par l'ajout par exemple d'alcali de type soude ou potasse. Dand ce cas particulier, l'acide itaconique est obtenu en mélange avec l'un de ses sels inorganiques. Il peut par exemple s'agir du sel de sodium ou de potassium.

Le glycérol peut être introduit dans le milieu nutritif de plusieurs manières. Classiquement, il est directement introduit, en mélange ou non avec un autre carbohydrate, dans le milieu nutritif. Toutefois, on peut également envisager le cas où le glycérol est déjà mis en oeuvre à titre de substrat carboné primaire dans la préparation de l'inoculum ou de la pré-culture du microorganisme sélectionné. Cet inoculum est dans une étape subséquente, transféré au sein d'un milieu nutritif pour initier la fermentation. Dans cette hypothèse, la concentration en glycérol, mélangé ou non à un autre carbohydrate, est complétée au niveau du milieu nutritif pour atteindre la concentration requise, précédement exposée.

A titre de micro-organismes utilisables selon l'invention on mentionnera tout particulièrement les espèces Aspergillus terreus et Aspergillus itaconicus.

Il s'agit plus particulièrement d'Aspergillus terreus et de préférence de la souche NRRL 1960 identifiée dans le brevet EP 341 112.

Outre le glycérol revendiqué selon l'invention, le milieu nutritif contient également les autres éléments nutritifs nécessaires à la fermentation. Il est bien entendu à la portée de l'homme de l'art de sélectionner ces autres éléments.

La source d'azote peut notamment être choisie parmi les composés organiques ou minéraux métabolisables, tels que l'extrait soluble de maïs (C.S.L.) et/ou de soja. l'urée, le sulfate d'ammonium, chlorure d'ammonium, phosphate d'ammonium, nitrate d'ammonium, etc ... et leurs mélanges.

Le milieu contient en outre des sels minéraux tels que sulfates, chlorures, phosphates, de Ca, Mg, Na, K, Fe, N, Co, Cu, Mn, Zn ainsi que d'autres additifs usuels tels que les agents de contrôle de pH et/ou des agents anti-moussants.

Le micro-organisme est introduit dans le milieu de fermentation de manière conventionnelle en soi à l'aide d'inoculum ou de cultures intermédiaires.

La fermentation est convenablement réalisée à un pH acide variant entre environ 1,8 et 5 et à une température d'environ 20° à environ 40°C, les conditions optimales dépendant de la souche particulière du micro-organisme employé.

Il est clair que la présente invention vise l'emploi du glycérol à titre de substrat carboné, unique ou non. dans un procédé de préparation d'acide itaconique et que sa portée s'étend à tout perfectionnement ou mode de réalisation particulier du procédé tel que revendiqué.

L'exemple 1 présenté çi-après à titre non limitatif de la présente invention permettra de mettre en évidence d'autres avantages de celle-ci.

### EXEMPLE 1

On réalise la production d'acide itaconique sur plusieurs milieux nutritifs se différenciant par la quantité de glycérol mise en oeuvre.

La solution nutritive utilisée contient 0,5 g d'extrait de maïs (CSL), 1,2 de nitrate d'ammonium, 0,3 g de sulfate de magnésium hydraté, 0,3 g d'oxyde de magnésium, 0,315 g d'hydroxyde de calcium, 0,05 g de phosphate monopotassique et 0,380 g de nitrate de cuivre hydraté. Le volume total de ce milieu de production est ajusté à 1 litre avec de l'eau et son pH porté à une valeur de l'ordre de 2,8 à 3 au moyen d'une solution d'acide nitrique.

Selon l'essai réalisé, ce milieu nutritif est complété avec du glycérol, en mélange ou non, avec du saccharose. Les quantités respectives de chacun des substrats carbonés sont précisées dans le tableau ci-après.
Pour chaque essai, le milieu nutritif précédent est introduit dans un erlenmeyer de 500ml et ensemencé par une culture d'Aspergillus terreus NRRL 1960 de manière à obtenir une concentration de l'ordre de 5.10⁷ spores/ml dans le milieu. La température du milieu est régulée à 32°- 35°C.
La fermentation est stoppée après épuisement du sucre et lorsque l'acidité est maximale et stable.
Des échantillons de moûts sont prélevés à la fin de la fermentation pour évaluation de la teneur en acide itaconique, déterminée par chromatographie liquide à haute performance.

Le tableau I ci-après rend compte de la productivité d'acide itaconique pour chaque essai.
Les résultats obtenus démontrent que le glycérol s'avère un substrat hydrocarboné parfaitement assimilable par le micro-organisme Aspergillus terreus et donc approprié à la production d'acide itaconique avec des rendements satisfaisants.

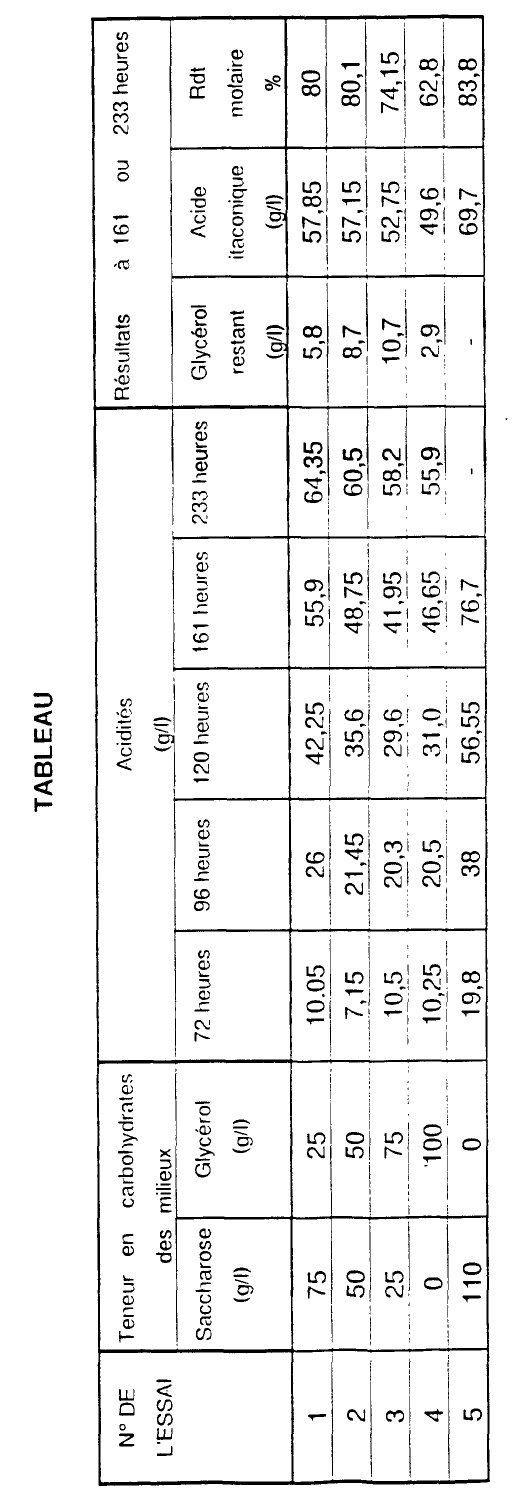

## Revendications

1. Procédé de production d'acide itaconique et/ou d'un de ses sels par fermentation aérobie d'un carbohydrate au moyen d'un micro-organisme **caractérisé en ce que** le micro-organisme est une souche d'Aspergillus terreus ou d'Aspergillus itaconicus, et en ce ledit procédé met en oeuvre un substrat carboné à base de glycérol et le cas échéant d'au moins un carbohydrate complémentaire, à une concentration variant entre 10 et 240 g/l exprimée en poids par volume.

2. Procédé selon la revendication 1 **caractérisé en ce que** le glycérol est introduit en mélange avec au moins un autre carbohydrate.

3. Procédé selon la revendication 2, **caractérisé en ce que** le carbohydrate complémentaire est choisi parmi les mono- et di-saccharides comme le glucose, le saccharose et le fructose, les amidons et les mélasses.

4. Procédé selon la revendication 1, **caractérisé en ce que** le micro-organisme est la souche Aspergillus terreus NRRL 1960.

## Patentansprüche

1. Verfahren zur Herstellung von Itaconsäure und/oder einem ihrer Salze durch aerobe Fermentation eines Kohlenhydrats mit einem Mikroorganismus, **dadurch gekennzeichnet, daß** der Mikroorganismus ein Stamm des Aspergillus terreus oder des Aspergillus itaconicus ist und **daß** das genannte Verfahren ein kohlenstoffhaltiges Substrat auf der Grundlage von Glycerin und gegebenenfalls von mindestens einem zusätzlichen Kohlenhydrat bei einer Konzentration verwendet, die zwischen 10 und 240 g/l, definiert als Gewicht pro Volumen, schwankt.

2. Verfahren nach Anspruch 1, **dadurch gekennzeichnet, daß** das Glycerin als Gemisch mit mindestens einem weiteren Kohlenhydrat zugegeben wird.

3. Verfahren nach Anspruch 2, **dadurch gekennzeichnet, daß** das zusätzliche Kohlenhydrat aus den Mono- und Disacchariden wie Glucose, Saccharose und Fructose, den Stärken und den Melassen gewählt ist.

4. Verfahren nach Anspruch 1, **dadurch gekennzeichnet, daß** der Mikroorganismus der Stamm Aspergillus terreus NRRL 1960 ist.

## Claims

1. A process for producing itaconic acid and/or one of its salts by aerobic fermentation of a carbohydrate using a micro-organism, **characterized in that** the micro-organism is a strain of Aspergillus terreus or Aspergillus itaconicus, and **in that** said process employs a carbon-containing substrate based on glycerol and, if appropriate, at least one complementary carbohydrate, in a concentration in the range 10 to 240 g/l expressed as the weight per volume.

2. A process according to claim 1, **characterized in that** the glycerol is introduced as a mixture with at least one other carbohydrate.

3. A process according to claim 2, **characterized in that** the complementary carbohydrate is selected from mono- and di-saccharides such as glucose, sucrose, fructose, starches or molasses.

4. A process according to claim 1, **characterized in that** the micro-organism is the Aspergillus terreus NRRL 1960 strain.
